# EUROPÄISCHE PATENTSCHRIFT

(11) **EP 2 204 242 B1**
(45) Veröffentlichungstag und Bekanntmachung des Hinweises auf die Patenterteilung: **07.02.2018**
(21) Anmeldenummer: 09015711.6
(22) Anmeldetag: 18.12.2009
(51) Int. Cl.: B08B 9/043, A61B 90/70

(54) **Vorrichtung zur Reinigung von Instrumentenkanälen**
Device for cleaning instrument channels
Dispositif de nettoyage de canaux d'instruments

(30) Priorität: 04.03.2009 DE 102009011204; 22.12.2008 DE 102008064205
(43) Veröffentlichungstag der Anmeldung: 07.07.2010
(73) Patentinhaber: Karl Storz SE & Co. KG, 78532 Tuttlingen (DE)
(72) Erfinder: Hansen, Norbert, Dr., 78576 Emmingen (DE)

(56) Entgegenhaltungen:
- EP-A2- 1 348 352
- EP-A2- 1 839 565
- WO-A1-2006/123941
- DE-B3-102006 022 366
- DE-U1- 20 012 968
- DE-U1-202007 000 793
- JP-A- 2006 051 057
- JP-A- 2008 284 317
- US-A- 5 964 004

## Beschreibung

Die vorliegende Erfindung beschreibt ein Verfahren und eine Vorrichtung zur Reinigung verunreinigter Instrumentenkanäle, insbesondere für die effiziente Reinigung von Kanälen in medizinischen Instrumenten.

Wird ein wieder verwendbares medizinisches Instrument am Patienten verwendet, so muss es nach dem Einsatz am Patienten gereinigt werden. Besonders aufwändig ist die Reinigung von Instrumentenkanälen medizinischer Produkte.

In der deutschen Patentanmeldung DE 2006 001 076 A1 wird eine Reinigungsbürste beschrieben, aufgebaut aus einem langen flexiblen Trägerelement und einem distal fixierten Bürstenkopf. Distal vom Bürstenkopf befindet sich noch ein überstehender Abschnitt des Trägerelements. Durch die Verlängerung des stabförmigen Trägerelements wird eine Verteilung von Flüssigkeiten durch eine schwingende oder federnde Bewegung des Trägerelements verhindert. Durch die Verteilung der Verunreinigungen im Instrumentenkanal durch die Bürste führt eine Reinigung mit dieser Reinigungsbürste zu keinem zufriedenstellenden Reinigungsergebnis.

In der Europäischen Patentanmeldung EP 1105061 B1 werden ein Verfahren und eine Vorrichtung zum Reinigen eines verunreinigten Lumens eines Endoskops beschrieben, durch welche die Verunreinigungen innerhalb des Instrumentenkanals gleichmäßig durch ein Ziehen und Schieben der Reinigungsvorrichtung im Instrumentenkanal verteilt werden. Dieser gleichmäßig im Kanal verteilte Film wird nachträglich durch eine enzymatische Reinigungsflüssigkeit behandelt. Die Reinigungsvorrichtung dient hier nicht der Entfernung der auszutragenden Substanzen aus dem Instrumentenkanal, sondern der gleichmäßigen Verteilung, um durch die nachträgliche Behandlung mit einer enzymatischen Reinigungsflüssigkeit zu verbessern. Die Reinigungswirkung ist nicht ausreichend.

Die Gebrauchsmusterschrift DE 20200700793 U1 zeigt einen Reinigungsdocht für einen Instrumentenkanal eines medizinischen Instruments. Hier wird ein Docht gezeigt, der in einem Teilbereich eine haftende Beflockung zeigt. Die Fasern bestehen aus Kunststoffmaterial oder sind Naturfasern. Bei Verwendung dieser Reinigungsvorrichtung wird die gesammelte Verunreinigung nach einmaliger Passage durch den Instrumentenkanal, beim Zurückziehen der Reinigungsvorrichtung im Instrumentenkanal verteilt. Eine nachträgliche Behandlung mit Reinigungsflüssigkeit oder eine erneute Reinigung, nach Reinigung der Fasern der Reinigungsvorrichtung, ist notwendig.

In der DE 10 2006 022 366 B3 wird ein Reinigungswerkzeug für einen Hörschlauch offenbart, das aus einem drahtartigem, flexiblen Element an dessen beiden Enden jeweils ein einziges Kunststoffendstück angebracht ist, offenbart. Durch eine Optimierung der Kunststoffendstücke durch umlaufende Kanten wird eine erhöhte Reinigungswirkung erzielt. Wird das Reinigungswerkzeug jedoch zur Reinigung des Hörschlauchs im Schlauch hin- und herbewegt, werden auch bei Verwendung dieser Vorrichtung die bereits gesammelten Verunreinigungen im Schlauch verteilt. Die Reinigung führt somit zu keinem ausreichenden Reinigungsergebnis.

Ein weiteres gattungsgemäßes Reinigungswerkzeug ist in der JP2006/051057 beschrieben.

Es ist die Aufgabe der vorliegenden Erfindung, ein Verfahren und eine Vorrichtung zur Reinigung von medizinischen Instrumentenkanälen zu zeigen, die ein effizientes und gutes Reinigungsergebnis erzielt.

Die vorliegende Erfindung löst diese Aufgabe durch die Reinigungsvorrichtung mit den Merkmalen des Anspruchs 1 bzw. ein Verfahren zur Reinigung mit den Merkmalen des Anspruches 14. Vorteilhafte Weiterbildungen sind in Unteransprüchen angegeben.

Die Reinigungsvorrichtung gemäß der vorliegenden Erfindung enthält ein stabförmiges Trägerelement mit Reinigungselementen. Die Reinigungselemente zeigen mindestens ein fixiertes Reinigungselement und zusätzlich wenigstens ein auf dem Trägerelement axial verschiebbares, lösbares Reinigungselement. Durch diesen mehrteiligen Aufbau der Reinigungselemente auf dem stabförmigen Trägerelement kann eine Verteilung der Verunreinigungen im Instrumentenkanal soweit als möglich reduziert werden. Die mehrfache Realisierung des Reinigungselements hat den Zweck ein distal zu einem fixierten Reinigungselement gelegenes lösbares Element, das durch das Vorschieben der Reinigungsvorrichtung durch den Instrumentenkanal den größeren Anteil an Verunreinigungen aufnimmt bzw. zusammenträgt, nach der typisch einmaligen Bewegung in axialer Richtung des Instrumentenkanals bis zu einem Endpunkt, vom Trägerelement abzulösen.

Bei nachfolgendem Zurückziehen der Reinigungsvorrichtung durch den Instrumentenkanal löst sich dieses lösbare Reinigungselement vom Trägerelement und ist am anschließend typischen Reinigungsvorgang nicht mehr beteiligt. Auf diese Weise wird eine Verteilung am insbesondere der am lösbaren Reinigungselement gebundenen Verunreinigungen im Instrumentenkanal reduziert, da ein großer Anteil der Verunreinigungen mit dem lösbaren Reinigungselement von der Reinigungsvorrichtung abgetrennt ist. Eine Reinigung mit der vorliegenden Reinigungsvorrichtung ist daher effizienter und reduziert die Verunreinigungen im bereits gereinigten Instrumentenkanal.

Durch das Ablösen des lösbaren Reinigungselements vom Trägerelement entsteht zusätzlich der Vorteil, dass beim Entfernen der Reinigungsvorrichtung aus dem Instrumentenkanal, das überstehende Trägerelement, auf welchem das distal gelegene lösbare Reinigungselement aufgesteckt ist, durch seinen Überstand ein unkontrolliertes Spritzen von möglicherweise verwendeten Reinigungsflüssigkeiten und dergleichen bei der Entnahme der restlichen Reinigungsvorrichtung aus dem Instrumentenkanal verhindert. Auf diese Weise kann eine Spritzkontamination der Umgebung mit eventuell infektiösem Material reduziert werden.

Die Begrifflichkeiten "lösbar" und "fixiert", wie vor - und nachstehend verwendet, zeigen unterschiedliche Ablösungs- und Befestigungsmöglichkeiten zwischen Reinigungselement und Trägerelement. Hierbei versteht man unter dem Begriff "lösbar" eine Art der Verbindung, die eine Verschiebung des Reinigungselements auf dem Trägerelement und damit ein Trennen vom Trägerelement, beispielsweise durch Abstreifen oder Herunterschieben des Reinigungselements vom Trägerelement, ermöglicht. Alternativ hierzu kann das Reinigungselement auch mit einem Teil des Trägerelements vom restlichen Trägerelement an einer vorgesehenen Trennstelle, beispielsweise durch ein Abbrechen an einer Sollbruchstelle oder durch Abschneiden an einer definierten Trennstelle, abgetrennt werden. Der Begriff "fixiert" stellt eine Verbindung dar, die keine Verschiebung des Reinigungselements auf dem Trägerelement aufgrund der axialen Bewegung im Instrumentenkanal ermöglicht. Die Verbindung zwischen dem Reinigungselement und dem Trägerelement ist während des Reinigungsverfahrens feststehend.

In einer besonderen Ausgestaltung ist mindestens ein Reinigungselement mit dem Trägerelement verbunden und ist ablösbar zum Trägerelement ausgebildet.

Dadurch wird eine erneute Verteilung der insbesondere am lösbaren Reinigungselement gebundenen Verunreinigungen im Instrumentenkanal reduziert, weil ein großer Anteil der Verunreinigungen durch die Ablösung des Reinigungselements von der Reinigungsvorrichtung abgetrennt wurde. Eine Reinigung ist äußerst effizient und reduziert die Verunreinigungen im bereits gereinigten Instrumentenkanal. Diese Ausgestaltung bietet des Weiteren den Vorteil, dass die Reinigungsvorrichtung selbst aufgrund der Ablösung des Reinigungselements vom Trägerelement eine hohe Reinigungsbeschaffenheit zeigt.

Eine Ausgestaltung der vorliegenden Erfindung zeigt, dass mindestens ein lösbares Reinigungselement distal am Trägerelement angeordnet ist und wenigstens ein fixiertes Reinigungselement proximal zu diesem angeordnet ist.

Dies bietet den Vorteil, dass beim Zurückziehen der Reinigungsvorrichtung sich das lösbare Reinigungselement vom Trägerelement ablösen kann und auf diese Weise die bereits angesammelten Verunreinigungen am lösbaren Reinigungselement nicht im bereits gereinigten Instrumentenkanal verteilt werden. Die Reinigung des Instrumentenkanals ist daher effizienter und ermöglicht ein besseres Reinigungsergebnis.

Vorzugsweise ist das lösbare Reinigungselement distal durch eine verschiebbare Verbindung am Trägerelement angebracht.

Es besteht zwar die Möglichkeit, das lösbare Reinigungselement nicht auf das Trägerelement aufzuschieben, sondern es mit dem Trägerelement und dem fixierten Reinigungselement durch den Instrumentenkanal zu schieben. Bevorzugterweise wird aber angestrebt das lösbare Reinigungselement mit einer Bohrung zu versehen, durch welche es auf das Trägerelement verschiebbar aufgesteckt werden kann. Dies ermöglicht ein gleichmäßiges Durchschieben des lösbaren Reinigungselements durch den Kanal und verhindert eine ungleichmäßige Verformung des Reinigungselements.

Es hat sich besonders bewährt, dass durch eine distal auf dem Trägerelement vorgesehene Trennstelle das Abtrennen eines Teils des Trägerelements mit mindestens einem Reinigungselement ermöglicht wird.

Diese Ausführung hat den Vorteil, dass das verunreinigte Reinigungselement unabhängig von seiner Formgebung, seinem Material und seinen Dehnungseigenschaften nach Austritt aus dem Instrumentenkanal vom Trägerelement gelöst werden kann und auf diese Weise die bereits zusammengetragenen Verunreinigungen nicht mehr im Instrumentenkanal verteilt werden. Die Reinigung von Instrumentenkanälen ist daher weitaus effizienter.

In einer besonderen Ausgestaltung sind mehrere fixierte Reinigungselemente auf dem stabförmigen Trägerelement befestigt und zeigen einen Zwischenraum zueinander.

Diese erfindungsgemäßen Zwischenräume bieten den Vorteil, dass innerhalb dieser Räume auch größere Ansammlungen von Verunreinigungen transportiert werden können, die nach einmaligem Passieren der Reinigungsvorrichtung durch den Instrumentenkanal mit dem lösbaren Reinigungselement abfallen bzw. manuell entfernt werden können. Des Weiteren ermöglichen sie eine Ausdehnung der Reinigungselemente in den Instrumentenkanal in axialer Richtung, was zu einem verbesserten Reinigungsergebnis führt.

Vorzugsweise ist das Trägerelement mit einem Anschlag versehen, der die Verschieblichkeit des Reinigungselements auf dem Trägerelement begrenzt.

Der für die Begrenzung der Verschieblichkeit des Reinigungselements vorgesehene Anschlag verhindert, dass sich das Reinigungselement vom Trägerelement während des Reinigungsvorgangs ablöst und im Instrumentenkanal stecken bleibt. Diese Art der Fixierung eines Reinigungselements auf dem Trägerelement bietet außerdem den Vorteil, dass sich der Anwender seine Reinigungsvorrichtung modular zusammenstellen kann, indem er beispielsweise die Eingrenzung der Verschieblichkeit der Reinigungselemente mit Kunststoffelementen, die er distal vom proximalen und/ oder distalen Ende des Reinigungselements anbringt, ermöglicht.

Es hat sich bewährt, dass der Bereich des Trägerelements, an dem ein Reinigungselement fixiert wird, durch das Zufügen oder Abtragen von Material die Eigenschaft aufweist, die den Zweck einer Fixierung der Reinigungselemente erfüllt. Beispielsweise kann das Auftragen einer metallischen Struktur auf den Bereich des Trägerelements den Durchmesser des Trägerelements an dieser Stelle vergrößern, so dass ein auf das stabförmige Trägerelement geschobenes Reinigungselement, das eine Bohrung mit dem Durchmesser des übrigen Trägerelements zeigt, fixiert wird. Eine Klebeverbindung stellt eine weitere bevorzugte Möglichkeit für die Befestigung des Reinigungselements am Trägerelement dar.

In einer bevorzugten Ausgestaltung der Fixierung eines Reinigungselements am Trägerelement sind Bereiche für die Fixierung am Trägerelement mit einem Gewinde versehen und damit fixiert, auf das die zu fixierende Reinigungselemente aufgeschraubt werden. Diese Ausgestaltung bietet den Vorteil, dass das Trägerelement nach seiner Anwendung wieder aufbereitet werden kann und, mit neuen Reinigungselementen versehen, wieder zur Reinigung von Instrumentenkanälen eingesetzt werden kann. Ein wiederaufbereitbares Trägerelement kann in seinen Materialeigenschaften und in seiner Ausgestaltung höherwertig gewählt werden, da es mehrmals für die Reinigung unterschiedlicher Kanäle eingesetzt werden kann. Die Kombination zwischen den Reinigungselementen als Einmalartikel und dem Trägerelement als wiederaufbereitbaren Artikel bietet daher wesentlich höherwertige Materialauslegung und Kostenreduzierung gleichermaßen. Das Material kann während des Reinigungsvorgangs durch die Verwendung von höherwertigen Materialien mehr beansprucht werden, was zu einem besseren und effizienteren Reinigungsergebnis führt.

Vorzugsweise sind die Reinigungselemente aus einem Material mit komprimierbaren Eigenschaften aufgebaut. Ein Material mit komprimierbaren Eigenschaften, bevorzugterweise mit filzartigen Eigenschaften, ermöglicht eine Kompression des Reinigungselements beim Einführen in den Instrumentenkanal. Auf diese Weise presst sich das Reinigungselement beim Vorschieben der Reinigungsvorrichtung an den Instrumentenkanal. Dies ermöglicht eine höhere abrasive Eigenschaft der Reinigungselemente. Es können dadurch beispielsweise auch größere Verunreinigungen, sowie am Instrumentenkanal haftende verhärtete Substanzen, besser entfernt werden. Durch die Verwendung des komprimierbaren Materials wird auch einem Verspritzen von Reinigungsflüssigkeit und gesammelten Verunreinigungen im Instrumentenkanal beim Austritt der Reinigungsvorrichtung aus dem Instrumentenkanal vorgebeugt, da sich die Reinigungselemente beim Austritt aufgrund des komprimierbaren Materials gleichmäßiger ausdehnen.

In einer besonders bewährten Ausgestaltung kann wenigstens ein Reinigungselement eine Flüssigkeit aufnehmen und abgeben.

Durch die Kombination der Reinigungsvorrichtung mit einer Flüssigkeit, insbesondere Wasser oder eine Flüssigkeit mit reinigenden Eigenschaften z.B. unter Verwendung von enzymatischen Substanzen, kann der Reinigungsprozess deutlich optimiert werden. Bevorzugterweise werden nicht alle Reinigungselemente mit einer Flüssigkeit versehen, so dass die übrigen Reinigungselemente auf dem Trägerelement die Flüssigkeiten aus dem Instrumentenkanal aufnehmen können.

In einer weiteren Ausgestaltung wird der Durchmesser der Reinigungselemente so gewählt, dass er größer ist als der kleinste und größte Durchmesser des zu reinigenden Instrumentenkanals.

Würden die Reinigungselemente einen geringeren Durchmesser als der kleinste und größte Durchmesser des Instrumentenkanals aufzeigen, so würde bei Vorschieben der Reinigungsvorrichtung durch den Instrumentenkanal in Axialrichtung die Reinigungselemente nicht kontinuierlich die Kanalinnenseite berühren und reinigen.

Ein größerer Durchmesser der Reinigungselemente als der kleinste und größte Durchmesser des Instrumentenkanals, welcher auch in seinem Durchmesser variieren könnte, verhindert daher eine Hin- und Herbewegung des Trägerelements quer zum Instrumentenkanal beim Reinigungsvorgang durch das Fachpersonal, da sich das Material der Reinigungselemente rundherum an die ganze Kanalwand anpresst. Auf diese Weise können vor allem Beschädigungen insbesondere durch das Trägerelement im Instrumentenkanal verhindert werden, da die Reinigungsvorrichtung gleichmäßig durch den Instrumentenkanal geführt wird und ein effizientes Reinigungsergebnis erzielt werden.

Besonders bewährt hat sich, dass die äußere Gestalt des Querschnitts der Reinigungselemente dem Querschnitt des zu reinigenden Instrumentenkanals entspricht.

Dies bietet den Vorteil, dass durch die gleiche Formgestaltung eine gleichmäßige Kompression des Materials der Reinigungselemente, sowie ein leichteres Einführen und Vorschieben der Reinigungsvorrichtung durch den Instrumentenkanal ermöglicht werden kann. Durch diese Maßnahme muss ein geringerer Kraftaufwand beim Vor- und Zurückbewegen der Reinigungsvorrichtung im Instrumentenkanal aufgebracht werden, der das Risiko einer Beschädigung innerhalb des Instrumentenkanals durch Fehlhandhabung deutlich reduziert und das Reinigungsverfahren für das Fachpersonal komfortabler gestaltet. Des Weiteren kann durch diese Ausgestaltung je nach Wahl des Verhältnisses der Abmessungen des Querschnitts von Instrumentenkanal und Reinigungselementen zueinander die abrasive Eigenschaft der Reinigungsvorrichtung variiert werden und den Reinigungsbedürfnissen angepasst werden.

In einer besonderen Ausführung der Reinigungsvorrichtung variiert der Grad der Härte der Reinigungselemente entlang des stabförmigen Trägerelements.

Durch diesen unterschiedlichen Grad der Härte, kann die Reinigungsvorrichtung sowohl zum Zusammentragen und Entfernen größerer Verunreinigungen, als auch zur Aufnahme von vorhandenen Flüssigkeiten, die sich vermehrt nach der Anwendung eines medizinischen Instruments am Patienten im Instrumentenkanal befinden, eingesetzt werden. Dabei nehmen die Reinigungselemente mit dem geringeren Härtegrad die im Instrumentenkanal vorhandenen Flüssigkeiten und Verunreinigungen auf, während die Reinigungselemente, deren Material einen höheren Härtegrad zeigen, durch ihre höhere abrasive Eigenschaften, größere und mehr anhaftende Substanzen vom Instrumentenkanal lösen und zusammentragen.

Gemäß einer besonders bevorzugten Ausgestaltung nehmen die Härteeigenschaften der Reinigungselemente auf dem Trägerelement von proximal nach distal ab.

In dieser besonderen Ausführung der Anordnung der Reinigungselemente mit unterschiedlichen Härteigenschaften liegt der Vorteil, dass die Reinigungsvorrichtung neben der Aufnahme von Flüssigkeiten und dem Zusammentragen von Verunreinigungen leichter in den Instrumentenkanal eingeführt werden kann. Die Anordnung der Reinigungselemente unterschiedlicher Härteigenschaften auf dem Trägerelement kann jedoch auch in umgekehrter Weise ansteigend sein.

In einer Ausgestaltung zeigt mindestens ein Reinigungselement auf dem Trägerelement einen Härtegradient des komprimierbaren Materials von proximal nach distal.

Dieser führt zu einem besonders guten Reinigungsergebnis auch wenn das Trägerelement nur mit wenigen Reinigungselementen bestückt ist, so können beispielsweise nur mit einem in axialer Richtung sehr langen Reinigungselement die Verunreinigungen im Instrumentenkanal effizient zusammengetragen werden.

Vorzugsweise zeigen die Reinigungselemente auf dem Trägerelement unterschiedliche Materialien.

Die Gestaltung der Reinigungselemente auf einem Trägerelement mit unterschiedlichen Materialien ermöglicht die Anpassung der Reinigungsvorrichtung an die unterschiedlichen Anforderungen, wie sie in der Praxis häufig auftreten. So kann beispielsweise für die Reinigung eines Spülkanals eine Vorrichtung gewählt werden, die Reinigungselemente aufweist, die aus einem elastischen Kunststoff bestehen, deren Kanten mit einer Abstreifkante versehen sind. Aber auch die Kombination von Reinigungselementen mit filzartigen Eigenschaften mit Reinigungselementen aus Kunststoff, beispielsweise aus einem schwammartigen Material, ist gerade bei der Verwendung von Reinigungsflüssigkeiten besonders wirkungsvoll.

In einer besonderen Ausgestaltung zeigen die Reinigungselemente auf einem Trägerelement unterschiedliche Durchmesser. In einer besonderen Ausführung hierzu sind die Durchmesser der Reinigungselemente auf dem Trägerelement von proximal nach distal abnehmend angeordnet.

Hierdurch wird das Einführen der Reinigungsvorrichtung in den Instrumentenkanal und damit die Handhabe vereinfacht, aber es werden auch durch die Abnahme der Durchmesser der Reinigungselemente von proximal nach distal die Reinigungselemente von proximal nach distal abnehmend unterschiedlich stark komprimiert, so dass durch diese Ausführung die stark komprimierten Reinigungselemente eine stärkere zusammentragende oder abrasive Funktion und die weniger komprimierten Reinigungselemente eine eher aufnehmende Funktion zeigen. Die Anordnung der unterschiedlichen Durchmesser der Reinigungselemente kann jedoch auch variieren oder auch von distal nach proximal abnehmend gewählt sein.

Besonders bewährt hat sich, die Gleitfähigkeit des distal gelegenen Reinigungselementes auf dem Trägerelement zu optimieren, indem der distal zum fixierten Reinigungselement gelegene Bereich des Trägerelements vorzugsweise mit einer besonders gleitfähigen Oberfläche, beispielsweise mit Teflon, beschichtet, behandelt oder aus einem entsprechenden reibungsarmen Material ausgebildet wird.

Das formstabile stabförmige Trägerelement kann aber auch vollständig mit einem reibungsarmen Kunststoff, beispielsweise Polytetrafluorethylen (PTFE), oder mit einem anderen gleitfähigen Material, beispielsweise Teflon, beschichtet werden.

Eine solche reibungsarme Ausgestaltung der Oberfläche des stabförmigen Trägerelements erleichtert die Ablösung des lösbaren Reinigungselements vom Trägerelement nach einmaliger Passage durch den Instrumentenkanal und verhindert bei Kontakt des Trägerelements mit dem zu reinigenden Instrumentenkanal zudem Beschädigungen am Kanal des Instruments.

Es hat sich vorteilhafterweise bewährt für das Trägerelement ein formstabiles Material, insbesondere Stahl zu wählen.

Unter dem Begriff "formstabil", wie vorstehend verwendet, versteht sich ein Material, das eine hohe Knicksteifigkeit und Stabilität aufweist und nicht deformierbar ist. Dies ermöglicht eine sehr verlässliche und sichere Reinigung von insbesondere geraden starren Endoskopen. Je nach Größe des Querschnitts kann das Material auch elastische Eigenschaften aufweisen oder aus einem elastischen Material aufgebaut sein, damit sich die Reinigungsvorrichtung dem Verlauf des Instrumentenkanals gut anpassen und damit auch für die Reinigung von flexiblen und ggf. gekrümmten medizinischen Instrumenten eingesetzt werden. Eine weitere Möglichkeit ist die Segmentierung des stabförmigen Trägerelements, um unterschiedliche Bereiche mit elastischen und deformierbaren Eigenschaften zu erzielen und damit besonders effizient schwierige Kanalgeometrien reinigen zu können.

In einer bevorzugten Ausführungsform ist das Trägerelement aus einem Vollmaterial gebildet.

Besteht das stabförmige Trägerelement aus Vollmaterial, so kann es trotz geringer Querschnittsflächen, die bei der Reinigung der Kanäle kleiner Instrumente mit geringem Durchmesser, häufig Verwendung finden, eine sehr hohe Stabilität und Knicksteifigkeit aufweisen. Das Vollmaterial bietet vor allem auch den Vorteil, dass sich keine Verschmutzungen im Trägerelement ansammeln kann, die wiederum, wenn das Trägerelement als wieder aufbereitetes Produkt realisiert wird, mit einer Reinigungsvorrichtung gereinigt werden müssen.

Besonders bewährt hat sich, dass das Trägerelement an seinen Enden eine Phase aufweist.

Eine Phase an den Enden des Trägerelements bietet den Vorteil, dass Beschädigungen, die durch die Reinigungsvorrichtung im Instrumentenkanal beispielsweise durch ein Entlangschrammen an der Kanaloberfläche verursacht werden können, vorgebeugt werden kann und dass zudem die Einführung der Reinigungsvorrichtung in den Instrumentenkanal unterstützt wird.

Vorzugsweise ist auf dem Trägerelement eine Markierung vorgesehen, die ein Maß für die Einschubtiefe der Reinigungsvorrichtung in den Instrumentenkanal darstellt und die gewünschte Endposition der Reinigungsvorrichtung im Instrumentenkanal repräsentiert.

Dies bietet den Vorteil, dass alle Teile des Instrumentenkanals verlässlich gereinigt werden und die Markierung ermöglicht dem Anwender den Instrumentenkanal vollständig zu reinigen ohne die Vorrichtung unnötig weit distal aus dem Kanal zu bewegen. So kann das lösbare Reinigungselement entsprechend der Position der Markierung beispielsweise nur soweit aus dem Instrumentenkanal geschoben werden, dass ein Teil des Reinigungselements noch im Instrumentenkanal steckt und so fixiert ist, dass es jedoch manuell aus dem Kanal entfernt werden kann. Das lösbare Reinigungselement fällt auf diese Weise nicht unkontrolliert aus dem Instrumentenkanal, eine unkontrollierte Verteilung von aufgenommen Substanzen in die Umgebung durch das lösbare Reinigungselement wird verhindert. Alternativ kann die Position der Markierung auch so gewählt sein, dass das lösbare Reinigungselement distal ganz aus dem Kanal austritt und sich beim Zurückziehen ablöst. Die Handhabung der Reinigungsvorrichtung ist daher durch diese Markierung weiterhin verbessert.

Es hat sich als vorteilhaft bewährt für die Reinigungsvorrichtung einen Behälter zur Aufnahme von kontaminierten Reinigungselementen mit einer verschließbaren Öffnung vorzusehen. Durch die Öffnung kann wenigstens ein Reinigungselement der Reinigungsvorrichtung in den Behälterinnenraum gebracht werden. Zusätzlich ist erfindungsgemäß der Behälter mit einer Vorrichtung zum Lösen von mindestens einem Reinigungselement von einem Trägerelement im Behälterinnenraum versehen.

Der Behälter zur Aufnahme von kontaminierten Reinigungselementen bietet den Vorteil, dass die Reinigungselemente nicht manuell nach einmaliger Passage durch den Instrumentenkanal und damit nach dem Austritt aus dem Instrumentenkanal vom Trägerelement abgelöst werden müssen, sondern durch eine Vorrichtung im Behälterinnenraum vom Trägerelement insbesondere automatisiert abgelöst werden und gemeinsam mit dem Behälter der Entsorgung zugeführt werden können, ohne dass eine Kontaminierung der Umgebung stattfindet. Dabei kann der Behälter sowohl als Einmalprodukt mit nur einem aufgenommenen Reinigungselement oder als auch Mehrwegprodukt nach der Aufnahme von mehreren Reinigungselementen in einem Abfallbehälter zur Ansammlung mehrere kontaminierter Reinigungselemente realisiert sein. Durch den Einsatz eines solchen Behälters wird das Risiko einer Kontamination gerade des Reinigungspersonals durch verunreinigte und verkeimte Reinigungselemente erheblich erniedrigt werden. Des Weiteren kann erfindungsgemäß einer weiteren Kontamination des Instrumentenkanals durch das Zurückziehen der verunreinigten Reinigungselemente durch den Instrumentenkanal vorgebeugt werden.

In einer besonderen Ausgestaltung wird mindestens ein Reinigungselement vom Trägerelement der Reinigungsvorrichtung durch eine Vorrichtung zum Lösen im Behälter an einer vorgesehenen Trennstelle, die beispielsweise als eine Sollbruchstelle oder eine Verjüngung des Trägerelements ausgebildet ist, abgelöst.

Die Vorrichtung zum Ablösen der Reinigungselemente vom Trägerelemente kann verschiedene Mechanismen zur Ablösung der verunreinigten Reinigungselemente aufweisen. In einer besonderen Ausgestaltung wird mindestens ein Reinigungselement durch Abzwicken gemeinsam mit einem Teil des Trägerelements von der Reinigungsvorrichtung abgelöst. Für diese besondere Ausführung kann auch eine Sollbruchstelle auf dem Trägerelement vorgesehen sein, die durch ein Abknicken der Reinigungsvorrichtung durch die Vorrichtung zum Lösen getrennt werden kann. Aber auch ein Abdrehen oder Abziehen des Reinigungselements vom Trägerelement ist denkbar. In dieser Ausgestaltung würde das Trägerelement in seiner ursprünglich geometrischen Form und in seiner Länge bestehen bleiben. All diese Mechanismen zur Ablösung der verunreinigten Reinigungselemente vom Trägerelement im Behälterinnenraum bieten jedoch den Vorteil, dass das Trägerelement nach dem Zurückziehen durch den Instrumentenkanal nach einem Aufbereitungsprozess, wieder mit neuen Reinigungselementen versehen werden kann und für einen neuen Reinigungsprozess eingesetzt werden können und dass eine Rekontamination des Instrumentenkanals durch Verunreinigungen und Keime erheblich reduziert wird.

Es hat sich als vorteilhaft erwiesen, dass der Behälter zur Aufnahme kontaminierter Reinigungselemente der Reinigungsvorrichtung einen Sensor aufweist, der die Position des zu lösenden Reinigungselements im Behälterinnenraum erfasst und der Vorrichtung zum Lösen signalisiert und diese das erfasste Reinigungselement vom Trägerelement durch einen Lösemechanismus automatisch ablöst.

Der Sensor zur Erfassung der Position der Reinigungsvorrichtung im Behälterinnenraum hat den Vorteil, dass die verunreinigten und verkeimten Reinigungselemente an der dafür vorgesehenen Trennstelle vom Trägerelement sicher und vollständig abgelöst werden. Auf diese Weise kann sehr sicher verhindert werden, dass ein verunreinigtes Reinigungselement nur teilweise oder überhaupt nicht vom Trägerelement abgelöst werden kann und beim Zurückziehen der des Trägerelements durch den Instrumentenkanal diesen wieder verunreinigt. In einer besonderen Ausgestaltung signalisiert der Sensor dem Anwender die genaue Position zur Ablösung des verunreinigten Reinigungselements von der Reinigungsvorrichtung, so dass der Anwender die Reinigungsvorrichtung ausreichend weit ins Behälterinnere für den Ablöseprozess der Reinigungselemente vom Trägerelement einführt. Auf diese Weise wird eine sichere Ablösung der Reinigungselemente aufgrund der erfassten und signalisierten Position im Behälterinnenraum gewährleistet.

Ein bevorzugtes Verfahren zur Reinigung von Instrumentenkanälen medizinischer Instrumente mit der vorstehend beschriebenen Reinigungsvorrichtung umfasst nachfolgend beschriebenen Ablauf.

Die Reinigungsvorrichtung wird nach dem Einführen in den zu reinigenden Instrumentenkanal in Axialrichtung im Instrumentenkanal bis zu einem Endpunkt bewegt, wobei nach Erreichen des Endpunkts die Reinigungsvorrichtung in entgegengesetzte axiale Richtung im Instrumentenkanal bewegt wird. In diesem Zusammenhang wird das lösbare Reinigungselement vom Trägerelement abgelöst und es ist anschließend am Reinigungsvorgang des bisher gereinigten Instrumentenkanals nicht mehr beteiligt.

Auf diese Weise wird eine Verteilung, insbesondere der am lösbaren Reinigungselement gebundenen Verunreinigungen im Instrumentenkanal reduziert, da ein regelmäßig großer Anteil der Verunreinigungen mit dem lösbaren Reinigungselement von der Reinigungsvorrichtung abgetrennt ist. Eine Reinigung mit der vorliegenden Reinigungsvorrichtung ist daher effizienter und reduziert die verbleibenden Verunreinigungen im Instrumentenkanal.

Unter Begriff "Endposition", wie vorstehend und nachstehend verwendet, versteht sich der Umkehrpunkt der Bewegung zwischen Vorschieben in axialer Richtung und Zurückziehen in entgegengesetzter axialer Richtung der Reinigungsvorrichtung durch den Instrumentenkanal. Die Endposition der Reinigungsvorrichtung kann so gewählt werden, dass das lösbare Reinigungselement am Ende des Instrumentenkanals austritt, und es in einem weiteren Arbeitsschritt manuell entfernt wird. Eine weitere Variante ist, dass die Reinigungsvorrichtung bereits vor dem Austritt des lösbaren Reinigungselements aus dem distalen Ende des Instrumentenkanals zurückgezogen wird. In diesem Fall befindet sich das lösbare Reinigungselement nach dem Reinigungsvorgang im Instrumentenkanal und wird in einem weiteren Schritt mit einer zusätzlichen Vorrichtung aus dem Instrumentenkanal geschoben. Hierfür kann alternativ ein Stab oder ein Trägerelement mit oder ohne Reinigungselemente genutzt werden. In einer bevorzugten Ausführung wird die Endposition der Reinigungsvorrichtung jedoch so gewählt, dass die Reinigungsvorrichtung so weit in den Instrumentenkanal vorgeschoben wird, dass das lösbare Reinigungselement vollständig aus dem distalen Ende des Instrumentenkanal austritt. Beim Austritt aus dem Instrumentenkanal dehnt sich das Reinigungselement aufgrund seiner insbesondere komprimierbaren Materialeigenschaften aus und fällt beim Zurückziehen der Reinigungsvorrichtung durch das Anschlagen an den zu reinigenden Instrumentenkanal ab. Auf diese Weise ist das lösbare Reinigungselement nicht mehr verschiebbar auf das Trägerelement aufgesteckt und daher nicht mehr am Reinigungsvorgang beteiligt. Das Einbringen der am abgelösten Reinigungselement anhaftenden Verunreinigungen ist damit erfindungsgemäß verhindert.

In einer besonderen Ausgestaltung wird die Reinigungsvorrichtung so weit in den Instrumentenkanal vorgeschoben, bis mindestens ein Reinigungselement vollständig aus dem distalen Ende des Instrumentenkanals austritt. Nach dem Austritt der Reinigungsvorrichtung aus dem Instrumentenkanal wird ein Teil des Trägerelements mit dem verunreinigten Reinigungselement an einer hierfür vorgesehenen Stelle abgetrennt. Auf diese Weise ist das Reinigungselement nicht mehr am Reinigungsvorgang beteiligt. Das Einbringen der am abgelösten Trägerelement mit dem Reinigungselement anhaftenden Verunreinigungen ist damit erfindungsgemäß verhindert.

In einem bevorzugten Verfahren zur Reinigung von Instrumentenkanälen wird mindestens ein zu lösendes Reinigungselement mit der Reinigungsvorrichtung nach dem Austritt der Reinigungsvorrichtung aus dem distalen Ende des Instrumentenkanals in einen Behälter zur Aufnahme von kontaminierten Reinigungselementen eingebracht. Im Innenraum des Behälters wird durch das Einbringen der Reinigungsvorrichtung ein Mechanismus ausgelöst, welcher das kontaminierte und verschmutzte Reinigungselement vom Trägerelement ablöst und nach der Entnahme des Trägerelements der Reinigungsvorrichtung aus dem Behälter dieser steril verschlossen wird.

Zusammenfassend zeigt die vorliegende Erfindung in anderen Worten ein Gerät und einen Prozess zur Entfernung von Verunreinigungen aus den Lumen medizinischer Instrumente.

Sie ist aus einem länglichen Basiselement und mindestens einem mit diesem Basiselement verbundenen Reinigungsglied aufgebaut und dadurch gekennzeichnet, dass mindestens ein Reinigungsglied sich beim Hin- und Herbewegen der Reinigungsvorrichtung im länglichen Lumen vom Basiselement abtrennt. Der Prozess zur Säuberung von Lumen medizinischer Instrumente mit dem erfindungsgemäßen Gerät umfasst folgende Schritte:
Das Gerät zur Säuberung der Lumen medizinischer Instrumente wird in Längsrichtung des Lumens bis zu einem Umkehrpunkt vorgeschoben. Nach Erreichen des Umkehrpunkts wird das Gerät zur Säuberung in die entgegengesetzte Richtung durch das Lumen des Instruments zurückbewegt. Durch die Zurückbewegung des Geräts zur Säuberung wird das verschiebbare, bewegliche Reinigungsglied vom Basiselement abgetrennt und ist anschließend am Säuberungsprozess des bisher gereinigten Instrumentenlumens nicht mehr beteiligt und kann dieses Lumen nicht mehr verschmutzen.

Ein Behältnis nimmt die kontaminierten und verschmutzten Reinigungsglieder auf und gewährleistet eine sichere Beseitigung der verschmutzten Reinigungsglieder.

Die vorliegende Erfindung zeigt ein Gerät und einen Prozess zur Säuberung von Lumen medizinischer Instrumente, die zu entfernende Substanzen aus dem Instrumentenlumen sicher heraus tragen und entsorgen und die Anforderung einer effizienten und guten Säuberung des Lumens erfindungsgemäß ermöglichen.

Weitere Vorteile der vorliegenden Erfindung sind aus nachfolgenden Figuren mehrerer bevorzugter Ausführungsbeispiele entnehmbar. Die Erfindung ist nicht auf diese Ausführungsbeispiele begrenzt. Hierbei werden gezeigt:
- Fig. 1: zeigt eine beispielhafte erfindungsgemäße Reinigungsvorrichtung mit einem stabförmigen Trägerelement und Reinigungselementen
- Fig. 2: zeigt eine Phase des erfindungsgemäßen Reinigungsverfahren mit der erfindungsgemäßen Reinigungsvorrichtung zu Begin des Reinigungsverfahrens
- Fig. 3: zeigt eine Phase des erfindungsgemäßen Reinigungsverfahrens mit der Reinigungsvorrichtung in der Endposition
- Fig. 4: zeigt eine Phase des erfindungsgemäßen Reinigungsverfahrens mit der erfindungsgemäßen Reinigungsvorrichtung nach Erreichen des Endpunkts und darauffolgendem Zurückziehen der Reinigungsvorrichtung
- Fig. 5: zeigt eine beispielhafte erfindungsgemäße Reinigungsvorrichtung mit einem stabförmigen Trägerelement und einem fixierten Reinigungselement nach dem erfindungsgemäßen Reinigungsvorgang
- Fig. 6: zeigt eine beispielhafte Realisierung eines stabförmigen Trägerelements für eine erfindungsgemäße Reinigungsvorrichtung
- Fig. 7: zeigt eine weitere Variante der erfindungsgemäßen Reinigungsvorrichtung mit beabstandet zueinander fixierten Reinigungselementen
- Fig. 8: zeigt eine Variante der erfindungsgemäßen Reinigungsvorrichtung mit mehreren am Trägerelement fixierten Reinigungselementen unterschiedlicher Durchmesser
- Fig. 9: zeigt eine Variante der erfindungsgemäßen Reinigungs-Vorrichtung mit einer besonderen Befestigungsmöglichkeit des Reinigungselements
- Fig.10: zeigt eine Variante der erfindungsgemäßen Reinigungsvorrichtung mit einem Behälter zur Aufnahme und Entsorgung kontaminierter Reinigungselemente
- Fig.11: zeigt beispielhaft die erfindungsgemäße Reinigungsvorrichtung mit einem am Trägerelement fixierten Reinigungselement in einer Vorrichtung zum Lösen eines Reinigungselements

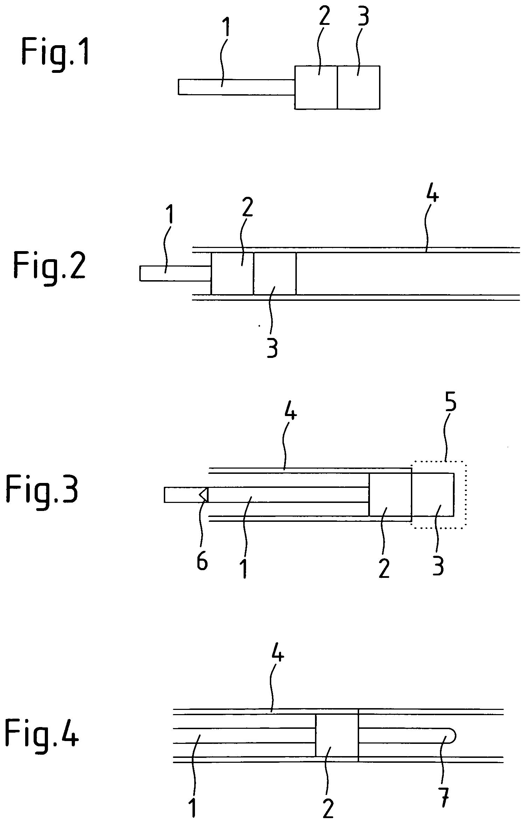

Die in Fig. 1 dargestellte Reinigungsvorrichtung zeigt ein Trägerelement 1 aus einem formstabilen, knicksteifen und nicht deformierbaren Material mit einem auf dem Trägerelement 1 fixierten Reinigungselement 2 und einem auf dem Trägerelement 1 axial verschiebbarem, lösbarem Reinigungselement 3.

Das Trägerelement 1 ist aus Vollmaterial, beispielsweise aus Stahl gebildet und hat einen kleineren Durchmesser als der zu reinigende Instrumentenkanal 4. Das Trägerelement 1 ist länger als der zu reinigende Instrumentenkanal 4 ausgeführt, damit die Reinigungsvorrichtung noch ausreichend gut mit der Hand gehalten werden kann, wenn die Reinigungsvorrichtung vollständig durch den Instrumentenkanal 4 in axialer Richtung zu der Endposition geschoben wird. Die Reinigungsvorrichtung zeigt je nach der Länge des zu reinigenden Instrumentenkanals 4 eine Länge von 0,5m bis 3m und einen Durchmesser zwischen 0,1cm und 0,8cm.

Die Reinigungselemente 2 und 3 zeigen einen größeren Durchmesser als der kleinste Durchmesser des zu reinigenden Instrumentenkanals 4. Auf diese Weise wird verhindert, dass die Fachkraft bei der Reinigung des Instrumentenkanals 4 mit der erfindungsgemäßen Reinigungsvorrichtung eine Hin- und Herbewegung des Trägerelements 1 quer zum Instrumentenkanal 4 für eine effizienten Reinigung ausführen muss. Auf diese Weise können zudem Beschädigungen an der Eintrittsöffnung, in die die Reinigungsvorrichtung eingeführt wird, im Instrumentenkanal 4 verhindert werden, da die Reinigungsvorrichtung gleichmäßig durch den Instrumentenkanal 4 geführt wird.

Die Reinigungselemente 2 und 3 sind aus einem Material mit komprimierbaren Eigenschaften, aus Kunststoff oder aus Naturfaser aufgebaut. Die Struktur des Materials ist filzartig. Dies hat den Vorteil, dass das Reinigungselement 2 und 3 sowohl abrasive als auch aufnehmende Eigenschaften aufweist. Das lösbare Reinigungselement 3 ist durch eine Bohrung, deren Durchmesser größer als der Vollmaterialquerschnitt des Trägerelements 1 ist, auf das stabförmige Trägelement 1 aufgesteckt. Durch die Beschichtung der Oberfläche mit Teflon des distal vom fixierten Reinigungselement 2 gelegenen Bereichs des Trägerelements 1 ist die Gleitfähigkeit des lösbaren Reinigungselements 3 auf dem Trägerelement 1 erhöht und dadurch ein Lösen vom Trägerelement 1 erfindungsgemäß erleichtert.

Die Fig. 2, 3 und 4 zeigen das bevorzugte Verfahren zur Reinigung mit der erfindungsgemäßen Reinigungsvorrichtung.

Die Reinigungsvorrichtung mit einem fixierten Reinigungselement 2 in Fig. 2 zeigt auf dem Trägerelement axial verschiebbarem, lösbarem Reinigungselement 3, die in einen zu reinigenden Instrumentenkanal 4 eingeführt ist. Die Anordnung eines fixierten und eines lösbaren Reinigungselement auf einem Trägerelement 1 hat den Zweck, dass das lösbare Reinigungselement 3, das distal zum fixierten Reinigungselement 2 liegt, durch das Vorschieben der Reinigungsvorrichtung im Instrumentenkanal 4 mehr Verunreinigungen aufnimmt und zusammenträgt als das fixierte Reinigungselement.

Fig. 3 zeigt die Ablösung des Reinigungselements 2. Diese erfolgt an dem Endpunkt, der hier bei vollständigem Austritt des lösbaren Reinigungselements 3 aus dem Instrumentenkanal 4 erreicht ist. Hierfür ist das stabförmige Trägerelement 1 mit einer Markierung 6 versehen, die zeigt, bis zu welchem Punkt die Reinigungsvorrichtung durch die Reinigungsfachkraft in den Instrumentenkanal 4 eingeschoben werden soll.

Die Reinigungsvorrichtung in Fig. 4 zeigt kein lösbares Reinigungselement 3. Durch das Zurückziehen der Reinigungsvorrichtung durch den Instrumentenkanal 4 löst sich das lösbare Reinigungselement 3, das sich durch seine komprimierbaren Materialeigenschaften sich beim Austritt aus dem Instrumentenkanal ausdehnt, vom Trägerelement 1 und ist am weiteren Reinigungsvorgang nicht mehr beteiligt. Auf diese Weise wird eine Verteilung, insbesondere der am lösbaren Reinigungselement 3 gebundenen Verunreinigungen im Instrumentenkanal 4, reduziert. Die Reinigung ist damit sehr effektiv.

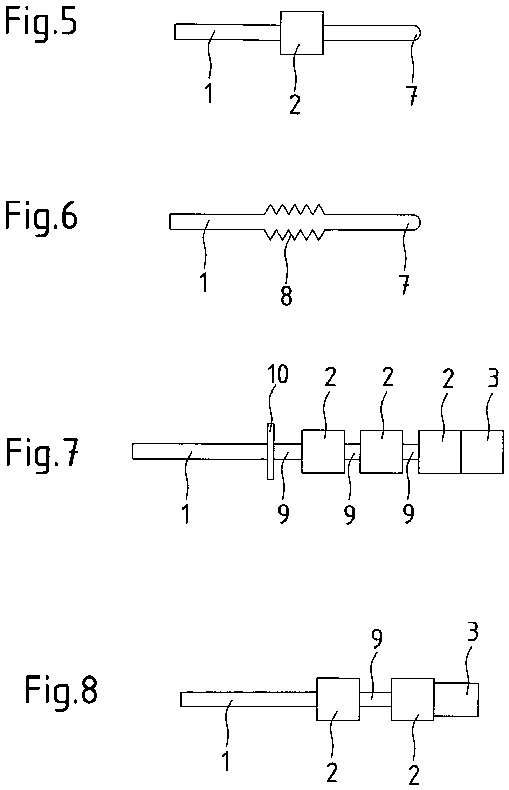

Fig. 5 zeigt die Reinigungsvorrichtung nach der Reinigung des Instrumentenkanals 4. Das lösbare Reinigungselement 3 hat sich aufgrund der Zurückbewegung der Reinigungsvorrichtung innerhalb des Instrumentenkanals vom Trägerelement 1 abgelöst. Es befindet sich ein fixiertes Reinigungselement 2 auf dem Trägerelement 1. Durch Ablösung des Reinigungselements 3 entsteht ein Überstand des Trägerelements 1 mit einem Abschnitt mit einer Phase 7 distal vom fixierten Reinigungselement 2, der ein Verspritzen von Reinigungsflüssigkeit und dergleichen nach Entfernung der Reinigungsvorrichtung aus dem Instrumentenkanal 4 reduziert.

Eine besondere Ausgestaltung der Befestigungsmöglichkeit eines fixierten Reinigungselements 2 wird in Fig.6 gezeigt. Das Trägerelement 1 zeigt einen Bereich, der mit einem Gewinde 8 versehen ist. Auf das Gewinde 8 wird das zu fixierende Reinigungselement 2 aus Filz aufgeschraubt. Hierzu zeigt das Reinigungselement 2 eine Bohrung, deren Durchmesser wenig größer als der Nenndurchmesser des Trägerelements 1 ist. Ein definiertes Lösen des fixierten Reinigungselements 2 vom Trägerelement 1 ist dadurch ermöglicht.

Dies bietet den Vorteil, dass das Trägerelement 1 nach seiner Anwendung wieder beispielsweise durch Sterilisierung aufbereitet werden kann und, mit neuen einmal zu verwendenden Reinigungselementen 2 und 3 versehen, wieder zur Reinigung von Instrumentenkanälen 4 eingesetzt werden kann. Durch die Wiederaufbereitung und die Wiederverwendung des Trägerelements 1 können Kosten deutlich reduziert werden. Das distale Ende des Trägerelements 1 ist abgerundet, um eine Beschädigung des Instrumentenkanals 4 zu vermeiden und ein einfacheres Einführen der Reinigungsvorrichtung in den Instrumentenkanal 4 zu ermöglichen.

Eine weitere besondere Anordnung der fixierten Reinigungselemente 2 auf dem stabförmigen Trägerelement 1 gemäß der Erfindung wird in Fig. 7 dargestellt. Zwischen den beiden fixierten Reinigungselementen 2 ist ein Zwischenraum 9.Dadurch wird ermöglicht, dass auszutragende Verunreinigungen darin angesammelt werden und nach der Passage durch den Instrumentenkanal 4, beim Austritt der Reinigungsvorrichtung aus dem Instrumentenkanal 4 abgefallen oder manuell entfernt werden. Auf diese Weise werden die Verunreinigungen, die sich im Zwischenraum 9 ansammeln, nicht beim Zurückziehen der Reinigungsvorrichtung durch den Instrumentenkanal 4 wieder in diesem verteilt. Diese Zwischenräume 9 erleichtern zudem die Einführung der Reinigungsvorrichtung, da die Reinigungselemente 2 und 3 aus einem komprimierbaren Material aufgebaut sind und einen größeren Durchmesser als der Instrumentenkanal 4 zeigen. Der Zwischenraum 9 bietet daher erfindungsgemäß Raum, in dem sich die Reinigungselemente 2 und 3 durch ihre Formveränderung beim Eintritt in den Instrumentenkanal 4 ausbreiten und sich auf diese Weise der Kanalgestalt gut anpassen können. Auf dem Trägerelement 1 sind mehrere Reinigungselemente 2 fixiert, die aus Filz bestehen. Sie haben daher sowohl abrasive, als auch aufnehmende Eigenschaften. Das proximal gelegene fixierte Reinigungslement 10 ist aus einem elastischen Kunststoff aufgebaut und ist zusätzlich mit einer Abstreifkante 10 versehen. Dies hat den Vorteil das Flüssigkeiten, die nicht vollständig durch die Reinigungselemente 2 und 3 erfasst oder aufgenommen werden, aus dem Instrumentenkanal 4 abgestreift werden.

In den bereits beschriebenen Figuren sind die Durchmesser aller Reinigungselemente 2 und 3 auf einem Trägerelemente 1 gleich groß. Fig. 7 zeigt eine alternative beispielhafte Ausführung der Reinigungsvorrichtung. Die Reinigungsvorrichtung in Fig. 7 zeigt mehrere Reinigungselemente 2 und ein lösbares Reinigungselement 3 mit unterschiedlichen Durchmessern. Die Durchmesser nehmen von proximal nach distal ab. Dies ermöglicht zum einen ein einfacheres Einführen der Reinigungsvorrichtung in den Instrumentenkanal 4 und zum anderen durch die variierende Komprimierung der Reinigungselemente 2 und 3 entstehen unterschiedliche Härtegrade der Reinigungselemente 2 und 3, die die abtragenden, zusammentragenden und aufnehmenden Funktionen der Reinigungsvorrichtung unterstützen, was zu einer besonders wirkungsvollen Reinigung führt.

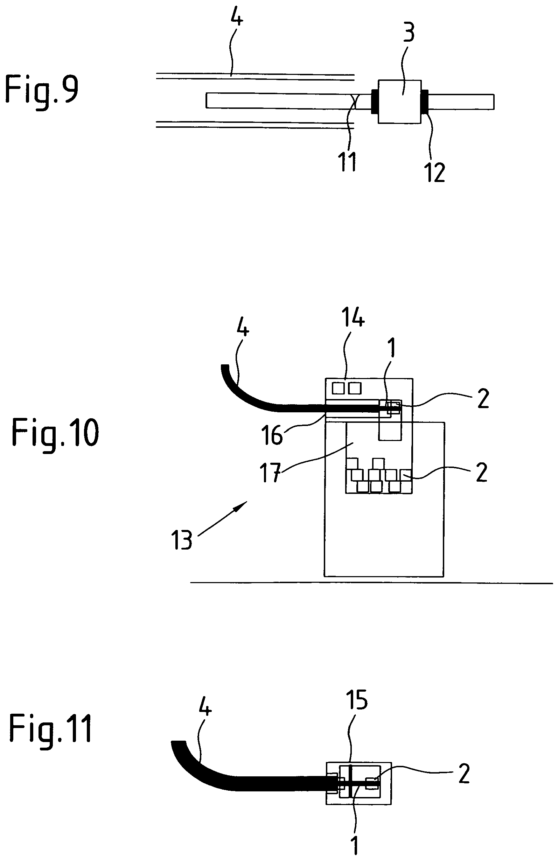

Eine alternative Ausgestaltung der Befestigungsmöglichkeit des Reinigungselements 3 am Trägerelement und der Ablösung nach dem Austritt aus dem Instrumentenkanal 4 zeigt Fig. 9. Das Reinigungselement 3 wird in seiner Verschieblichkeit auf dem Trägerelement 1 durch einen proximal und distal am Trägerelement 1 angebrachten Anschlag 12 gehemmt. Diese Anschläge 12 sind in diesem Ausführungsbeispiel aus Kunststoffringen ausgebildet, die rutschfest proximal und distal vom Reinigungselement 3 angebracht sind. Das Reinigungselement 3 wird nach dem distalen Austritt aus dem Instrumentenkanal 4 mit einer Schneidvorrichtung an der vorgesehenen Trennstelle 11 abgetrennt.

Dieser modulare Aufbau der Reinigungsvorrichtung bietet dem Anwender zahlreiche Möglichkeiten, eine Reinigungsvorrichtung für den individuellen Einsatz zur Reinigung medizinischer Instrumentenkanäle zusammenzustellen.

Fig. 10 zeigt eine erfindungsgemäße Reinigungsvorrichtung. Das Trägerelement 1 mit Reinigungselementen 3, welches das distale Ende des Instrumentenkanals 4 passiert hat, ist in den Behälterinnenraum des Behälters 13 zur Aufnahme kontaminierter Reinigungselemente 3 eingeführt.

Der Behälter 13 ist insbesondere aus Polyethylen aufgebaut, durch welches eine hohe Standfestigkeit des Behälters gewährleistet ist. Die Reinigungsvorrichtung wird mit dem Instrumentenkanal 4 des endoskopischen Systems in die Öffnung 16 des Behälters eingeführt. Die Öffnung 16 des Behälters ist mit einer Klappe versehen, die durch die Einführung der Reinigungsvorrichtung weggeklappt wird. Wenn keine Entsorgung von Reinigungselementen 3 stattfindet, stellt die Klappe an der Öffnung 16 sicher, dass der Behälter 13 keimfrei verschlossen ist und eine Kontaminierung auf das Reinigungspersonal verhindert wird. Im Behälterinnenraum ist ein entnehmbarer Abfallbehälter 17 vorgesehen, der die Ansammlung von Reinigungselementen 3 mehrerer Reinigungsvorrichtungen nach der Ablösung vom Trägerelement 1 ermöglicht.

Ein Sensor im Behälter 13 signalisiert durch das Sensorsignal eine mittels LED - Lampe 14, dem Anwender die richtige Position der Reinigungsvorrichtung in der Vorrichtung zum Lösen 15, so dass die Abtrennung durch ein Abnicken des Trägerelements 1 in der Vorrichtung 15 erfolgt, was zum Bruch der vorgesehenen Trennstelle 11 führt und das Reinigungselement 3 vom Trägerelement 1 abgelöst ist und in den Abfallbehälter 17 des Behälters zur Aufnahme kontaminierter Reinigungselemente 13 fällt. Dadurch können Reinigungselemente 3 unterschiedlicher Reinigungsvorrichtungen gemeinsam mit dem Abfallbehälter 17 entsorgt werden.

Fig. 11 zeigt eine weitere Variante zur Entsorgung kontaminierter Reinigungselemente 3. Die Vorrichtung zum Lösen 15 wird dabei manuell über ein kontaminiertes Reinigungselement 3, welches distalseitig aus dem endoskopischen System herausragt, gesteckt. Durch ein Zusammendrücken der Vorrichtung zum Lösen 15 wird das Reinigungselement 3 ergriffen und beim Zurückziehen des Trägerelement 1 abgelöst. Nach der anschließenden Entnahme des endoskopischen Systems aus der Vorrichtung 15 verschließt sich die Vorrichtung 15 keimfrei. Die Vorrichtung zum Lösen 15 wird zusammen mit dem aufgenommenen kontaminierten Reinigungselement 3 als eine Einheit entsorgt. Das Trägerelement 1, wird nach dem Lösen der kontaminierten Reinigungselemente 3 und der Entnahme aus dem Instrumentenkanal 4 wiederaufbereitet und kann wieder verwendet werden.

## Patentansprüche

1. Reinigungsvorrichtung für eine Reinigung von Kanälen medizinischer Instrumente mit einem stabförmigen Trägerelement (1) und mindestens ein mit dem Trägerelement (1) verbundenes Reinigungselement (2,3), **dadurch gekennzeichnet, dass** das Reinigungselement (2,3) vom Trägerelement (1) lösbar ist und mindestens ein Reinigungselement am Trägerelement (1) fixiert ist und mindestens ein zusätzliches Reinigungselement vom Trägerelement lösbar ist wobei, mindestens ein lösbares Reinigungselement (3) distal am Trägerelement (1) angeordnet ist und wenigstens ein fixiertes Reinigungselement (2) proximal zu diesem angeordnet ist und, dass das lösbare Reinigungselement (3) durch eine verschiebliche Verbindung an das Trägerelement (1) distal aufgebracht ist **dadurch gekennzeichnet, dass** das Trägerelement (1) einen Anschlag (12) aufweist, der die Verschieblichkeit des Reinigungselements (2,3) auf dem Trägerelement (1) begrenzt.

2. Reinigungsvorrichtung nach dem vorherigen Anspruch, **dadurch gekennzeichnet, dass** eine Trennstelle (11) distal am Trägerelement (1) vorgesehen ist, die ein Abtrennen eines Teils des Trägerelements (1) mit mindestens einem Reinigungselement (2,3) ermöglicht.

3. Reinigungsvorrichtung nach einem der oben genannten Ansprüchen, **dadurch gekennzeichnet, dass** das mindestens eine Reinigungselement (2,3) aus einem insbesondere komprimierbaren, filzartigen Material aufgebaut ist.

4. Reinigungsvorrichtung nach einem der vorherigen Ansprüche, **dadurch gekennzeichnet, dass** das Material wenigstens eines Reinigungselementes (2,3) eine Reinigungsflüssigkeit aufnehmen und abgeben kann.

5. Reinigungsvorrichtung nach einem der vorherigen Ansprüche, **dadurch gekennzeichnet, dass** der Grad der Härte der Reinigungselemente (2,3) entlang des Trägerelements (1) variiert.

6. Reinigungsvorrichtung nach einem der vorherigen Ansprüche, **dadurch gekennzeichnet, dass** wenigstens ein Reinigungselement (2,3) aus einem elastischen Kunststoff aufgebaut ist, der insbesondere mit einer Abstreifkante versehen sind.

7. Reinigungsvorrichtung nach den vorherigen Ansprüchen, **dadurch gekennzeichnet, dass** das Trägerelement (1) an einem oder beiden Enden eine Phase (7) aufweist.

8. Reinigungsvorrichtung nach einem der vorherigen Ansprüche, **dadurch gekennzeichnet, dass** das Material des Trägerelements (1) formstabil gewählt ist und insbesondere mit einem reibungsarmen Kunststoff, beispielsweise Polytetrafluorethylen (PTFE) beschichtet ist.

9. Reinigungsvorrichtung nach einem der vorherigen Ansprüche **gekennzeichnet durch** einen Behälter (13) zur Aufnahme von kontaminierten Reinigungselementen (3) mit einer verschließbaren Öffnung (16), durch die wenigstens ein Reinigungselement (3) der Reinigungsvorrichtung in den Behälterinnenraum gebracht werden kann, wobei der Behälter (13) mit einer Vorrichtung zum Lösen (15) von mindestens einem Reinigungselement (3) von dem Trägerelement (1) im Behälterinnenraum versehen ist.

10. Verfahren zur Reinigung von Instrumentenkanälen (4) medizinischer Instrumente mit einer Reinigungsvorrichtung, wobei das Verfahren folgende Schritte umfasst:
Bewegen der Reinigungsvorrichtung in Axialrichtung im Instrumentenkanal (4) bis zu einem Endpunkt, wobei nach Erreichen des Endpunkts die Reinigungsvorrichtung in entgegengesetzte axiale Richtung im Instrumentenkanal (4) hinaus bewegt wird und ein lösbares Reinigungselement (3) vom Trägerelement (1) abgelöst wird und anschließend am Reinigungsvorgang des bisher gereinigten Instrumentenkanals (4) nicht mehr beteiligt ist.

11. Verfahren nach Anspruch 10 , **dadurch gekennzeichnet, dass** die Reinigungsvorrichtung in axialer Richtung im Instrumentenkanal (4) über das Ende des Instrumentenkanal (4) bewegt wird, wobei mindestens ein Reinigungselement (3) vom Trägerelement (1) gelöst wird, so dass das Reinigungselement (3) beim Zurückziehen der Reinigungsvorrichtung in entgegengesetzte axiale Richtung am Reinigungsvorgang des Instrumentenkanals (4) nicht mehr beteiligt ist.

12. Verfahren nach Anspruch 11, **dadurch gekennzeichnet, dass** sich das lösbare Reinigungselement (3) nach dem Austritt aus dem Instrumentenkanal (4) dekomprimiert und beim Zurückziehen nicht wieder in den Instrumentenkanal (4) eingezogen wird und dadurch vom Trägerelement (1) abgelöst wird.

13. Verfahren nach einem der Ansprüche 10 bis 12, **dadurch gekennzeichnet dass** die Reinigungsvorrichtung in axialer Richtung im Instrumentenkanal (4) über das Ende des Instrumentenkanal (4) bewegt wird, wobei mindestens ein Reinigungselement (3) mit dem von der Trennstelle (11) distal gelegenen Abschnitt des Trägerelements (1) vom stabförmigen Trägerelement abgetrennt wird , so dass das Reinigungselement (3) mit dem distalen Abschnitt des Trägerelements beim Zurückziehen der Reinigungsvorrichtung durch den Instrumentenkanal (4) am Reinigungsvorgang nicht mehr beteiligt ist.

## Claims

1. Cleaning device for cleaning channels of medical instruments, with a rod-shaped carrier element (1) and at least one cleaning element (2, 3) connected to the carrier element (1), **characterized in that** the cleaning element (2, 3) is releasable from the carrier element (1), and at least one cleaning element is fixed on the carrier element (1), and at least one additional cleaning element is releasable from the carrier element, wherein at least one releasable cleaning element (3) is arranged distally on the carrier element (1), and at least one fixed cleaning element (2) is arranged proximally thereto, and **in that** the releasable cleaning element (3) is mounted distally on the carrier element (1) by a displaceable connection, **characterized in that** the carrier element (1) has a limit stop (12) which limits the displaceability of the cleaning element (2, 3) on the carrier element (1).

2. Cleaning device according to the preceding claim, **characterized in that** a separation point (11) is provided distally on the carrier element (1) and permits separation of a part of the carrier element (1) with at least one cleaning element (2, 3) .

3. Cleaning device according to either of the abovementioned claims, **characterized in that** the at least one cleaning element (2, 3) is constructed from an in particular compressible, felt-like material.

4. Cleaning device according to one of the preceding claims, **characterized in that** the material of at least one cleaning element (2, 3) can absorb and release a cleaning liquid.

5. Cleaning device according to one of the preceding claims, **characterized in that** the degree of hardness of the cleaning elements (2, 3) varies along the carrier element (1).

6. Cleaning device according to one of the preceding claims, **characterized in that** at least one cleaning element (2, 3) is constructed from a flexible plastic, which is provided in particular with a stripping edge.

7. Cleaning device according to the preceding claims, **characterized in that** the carrier element (1) has a bevel (7) at one or both ends.

8. Cleaning device according to one of the preceding claims, **characterized in that** the material of the carrier element (1) is chosen to be dimensionally stable and in particular is coated with a low-friction plastic, for example polytetrafluoroethylene (PTFE).

9. Cleaning device according to one of the preceding claims, **characterized by** a container (13) for receiving contaminated cleaning elements (3), with a closeable opening (16) through which at least one cleaning element (3) of the cleaning device can be brought into the container interior, wherein the container (13) is provided with a device for releasing (15) at least one cleaning element (3) from the carrier element (1) in the container interior.

10. Method for cleaning instrument channels (4) of medical instruments with a cleaning device, the method having the following steps: moving the cleaning device in the axial direction in the instrument channel (4) as far as an end point, wherein the cleaning device, after reaching the end point, is moved out in the opposite axial direction in the instrument channel (4) and a releasable cleaning element (3) is released from the carrier element (1) and then no longer takes part in the cleaning of the hitherto cleaned instrument channel (4).

11. Method according to Claim 10, **characterized in that** the cleaning device is moved in the axial direction in the instrument channel (4) beyond the end of the instrument channel (4), wherein at least one cleaning element (3) is released from the carrier element (1) such that, when the cleaning device is pulled back in the opposite axial direction, the cleaning element (3) no longer takes part in the cleaning the instrument channel (4).

12. Method according to Claim 11, **characterized in that** the releasable cleaning element (3) is decompressed after emerging from the instrument channel (4) and, upon being pulled back, is not drawn into the instrument channel (4) again and is thereby released from the carrier element (1).

13. Method according to one of Claims 10 to 12, **characterized in that** the cleaning device is moved in the axial direction in the instrument channel (4) beyond the end of the instrument channel (4), wherein at least one cleaning element (3), with the portion of the carrier element (1) situated distally from the separation point (11), is separated from the rod-shaped carrier element such that, when the cleaning device is pulled back through the instrument channel (4), the cleaning element (3) with the distal portion of the carrier element no longer takes part in the cleaning process.

## Revendications

1. Dispositif de nettoyage destiné au nettoyage des canaux des instruments médicaux avec un élément de support (1) en forme de tige et au moins un élément de nettoyage (2, 3), lequel est raccordé à l'élément de support (1), **caractérisé en ce que** l'élément de nettoyage (2, 3) peut être retiré de l'élément de support (1) et au moins un élément de nettoyage est fixé de manière stationnaire à l'élément de support (1) et au moins un élément de nettoyage supplémentaire peut être retiré de l'élément de support, selon lequel au moins un élément de nettoyage (3) amovible est disposé de manière distale au niveau de l'élément de support (1) et tout au moins un élément de nettoyage (2) fixé de manière stationnaire est disposé de manière proximale par rapport à celui-ci et **caractérisé en ce que** l'élément de nettoyage (3) amovible est mis en place de manière distale au niveau de l'élément de support (1) par l'intermédiaire d'une jonction coulissante et **caractérisé en ce que** l'élément de support (1) présente une butée (12), laquelle délimite la capacité de coulissement de l'élément de nettoyage (2, 3) sur l'élément de support (1).

2. Dispositif de nettoyage selon la revendication 1, **caractérisé en ce qu'**une zone de séparation (11) est prévue de manière distale au niveau de l'élément de support (1), laquelle permet une séparation d'une partie de l'élément de support (1) avec au moins un élément de nettoyage (2, 3).

3. Dispositif de nettoyage selon l'une des revendications susmentionnées, **caractérisé en ce que** la structure de l'au moins un élément de nettoyage (2, 3) est constituée à partir d'un matériau semblable à du feutre et qui, en particulier, peut être comprimé.

4. Dispositif de nettoyage selon l'une des revendications précédentes, **caractérisé en ce que** le matériau de tout au moins un élément de nettoyage (2, 3) peut absorber et dispenser un liquide de nettoyage.

5. Dispositif de nettoyage selon l'une des revendications précédentes, **caractérisé en ce que** le degré de dureté des éléments de nettoyage (2, 3) est variable le long de l'élément de support (1).

6. Dispositif de nettoyage selon l'une des revendications précédentes, **caractérisé en ce que** la structure de tout au moins un élément de nettoyage (2, 3) est constituée à partir d'un plastique élastique, lequel est pourvu, en particulier, d'une arête de raclage.

7. Dispositif de nettoyage selon les revendications précédentes, **caractérisé en ce que** l'élément de support (1) présente une phase (7) au niveau de l'une de ses extrémités ou de ses deux extrémités.

8. Dispositif de nettoyage selon l'une des revendications précédentes, **caractérisé en ce que** le matériau de l'élément de support (1) est sélectionné de telle sorte qu'il est stable de forme et, en particulier, est revêtu d'un plastique à faible coefficient de friction, par exemple du polytétrafluoroéthylène (PTFE).

9. Dispositif de nettoyage selon l'une des revendications précédentes, **caractérisé par** un réservoir (13) qui est destiné à la réception des éléments de nettoyage (3) contaminés et qui comprend une ouverture (16) pouvant être verrouillée, à travers laquelle le tout au moins un élément de nettoyage (3) du dispositif de nettoyage peut être inséré dans le compartiment intérieur du réservoir, selon lequel le réservoir (13) est pourvu, dans le compartiment intérieur du réservoir, d'un dispositif destiné à la séparation (15) de l'au moins un élément de nettoyage (3) avec l'élément de support (1).

10. Procédé destiné au nettoyage de canaux d'instrument (4) des instruments médicaux avec un dispositif de nettoyage, selon lequel le procédé comprend les phases suivantes : le déplacement du dispositif de nettoyage dans la direction axiale, à l'intérieur du canal des instruments (4), jusqu'à un point extrême, selon lequel, après avoir atteint le point extrême, le dispositif de nettoyage est déplacé vers l'extérieur dans la direction axiale opposée, à l'intérieur du canal des instruments (4), et un élément de nettoyage (3) amovible est séparé de l'élément de support (1), puis n'est plus impliqué dans le processus de nettoyage du canal des instruments (4) qui vient d'être nettoyé.

11. Procédé selon la revendication 10, **caractérisé en ce que** le dispositif de nettoyage est déplacé dans la direction axiale, à l'intérieur du canal des instruments (4), par l'extrémité du canal des instruments (4), selon lequel au moins un élément de nettoyage (3) est séparé de l'élément de support (1) de telle sorte que l'élément de nettoyage (3) n'est plus impliqué dans le processus de nettoyage du canal des instruments (4), quand le dispositif de nettoyage est retiré dans la direction axiale opposée.

12. Procédé selon la revendication 11, **caractérisé en ce que** l'élément de nettoyage (3) amovible est décomprimé après sa sortie en dehors du canal des instruments (4) et n'est plus réintroduit dans le canal des instruments (4) lors du retrait et se trouve par conséquent séparé de l'élément de support (1).

13. Procédé selon l'une des revendications 10 à 12, **caractérisé en ce que** le dispositif de nettoyage est déplacé par l'extrémité du canal des instruments (4) dans la direction axiale, à l'intérieur du canal des instruments (4), selon lequel au moins un élément de nettoyage (3) avec la section de l'élément de support (1) est séparé de l'élément de support en forme de tige, laquelle section est positionnée de manière distale par rapport à la zone de séparation (11), de telle sorte que l'élément de nettoyage (3) avec la section distale de l'élément de support n'est plus impliqué dans le processus de nettoyage quand le dispositif de nettoyage est retiré à travers le canal des instruments (4).
